# EUROPEAN PATENT APPLICATION

(11) **EP 4 364 845 A1**
(43) Date of publication of application: **08.05.2024**
(21) Application number: 22833333.2
(22) Date of filing: 01.07.2022
(51) Int. Cl.: B01J 29/46, B01J 37/16, C10L 3/12

(54) **CATALYST FOR LIQUEFIED PETROLEUM GAS SYNTHESIS AND METHOD FOR PRODUCING LIQUEFIED PETROLEUM GAS**

(30) Priority: 02.07.2021 JP 2021111070; 15.04.2022 JP 2022067957
(71) Applicant: FURUKAWA ELECTRIC CO., LTD., Tokyo 100-8322 (JP)
(72) Inventor: MORI Tomohiko, Tokyo 100-8322 (JP); FUJIKAWA Takashi, Tokyo 100-8322 (JP); IWANO Yuki, Tokyo 100-8322 (JP); BAMBA Yuichiro, Tokyo 100-8322 (JP); KAWAMATA Yuki, Tokyo 100-8322 (JP); FUKUSHIMA Masayuki, Tokyo 100-8322 (JP)
(74) Representative: Winter, Brandl - Partnerschaft mbB
(86) International application number: PCT/JP2022/026510
(87) International publication number: WO 2023/277188

(57) **Abstract**

A catalyst for synthesizing liquefied petroleum gas according to the present invention includes: a Cu-Zn-based catalytic material; and an MFI-type zeolite catalytic material supporting a noble metal, in which the MFI-type zeolite catalytic material contains P, and a ratio of mass (M_{P}) of P in the MFI-type zeolite catalytic material to mass (M2) of the MFI-type zeolite catalytic material is more than 0 mass% and less than 4.5 mass%.

## Description

### TECHNICAL FIELD

The present disclosure relates to a catalyst for synthesizing liquefied petroleum gas and a method for producing liquefied petroleum gas.

### BACKGROUND ART

Liquefied petroleum gas (LPG) mainly consisting of propane and butane exists mixed with impurity gases such as methane and ethane in oil fields and natural gas fields. After transferring such gases to aboveground facilities, propane and butane are separated and recovered from the gases, further, impurities such as sulfur and mercury are removed to obtain liquefied petroleum gas.

Additionally, liquefied petroleum gas is also contained in crude oil. Therefore, it is also possible to obtain liquefied petroleum gas by separating and extracting propane and butane during the refining process at oil refineries.

Moreover, methods for producing liquefied petroleum gas from synthesis gas of carbon monoxide and hydrogen have been studied. For instance, Patent Documents 1 to 4 disclose methods for producing liquefied petroleum gas in which the main component is butane.

Non-Patent Documents 1 and 2 disclose methods for producing hydrocarbons including isobutane as the main component from the synthesis gas of carbon monoxide and hydrogen.

In the aforementioned patent documents, liquefied petroleum gas and gasoline are primarily produced using a mixed catalyst of a methanol synthesis catalyst and zeolite supporting palladium.

In the liquefied petroleum gas produced according to the aforementioned patent documents, the content ratio of butane tends to be higher than that of propane. Given that butane is less volatile than propane, using butane as a fuel in cold regions is not easy. For this reason, it is preferable that the content ratio of propane to the combined total of propane and butane is high.

### Citation List

### Patent Document

Patent Document 1: Japanese Patent No. 5405103
Patent Document 2: Japanese Patent No. 4965258
Patent Document 3: Japanese Patent No. 4558751
Patent Document 4: Japanese Unexamined Patent Application, Publication No. 2019-37939

### Non-Patent Document

Non-Patent Document 1: Congming Li, Kaoru Fujimoto, "Synthesis gas conversion to iso-butane-rich hydrocarbons over a hybrid catalyst containing Beta zeolite - role of doped palladium and influence of the SiO2/Al2O3 ratio", Catalysis Science & Technology, 2015, 5, 4501-4510
Non-Patent Document 2: Congming Li, Hongyan Ban, Weijie Cai, Yu Zhang, Zhong Li, Kaoru Fujimoto, "Direct synthesis of iso-butane from synthesis gas or CO2 over CuZnZrAl/Pd-β hybrid catalyst", Journal of Saudi Chemical Society (2017)21, 974-982

### DISCLOSURE OF THE INVENTION

### Problems to be Solved by the Invention

The object of the present disclosure is to provide a catalyst for synthesizing liquefied petroleum gas and a method for producing liquefied petroleum gas, which can increase the ratio of propane to the combined total of propane and butane in the produced liquefied petroleum gas.

### Means for Solving the Problems

[1] A catalyst for synthesizing liquefied petroleum gas including a Cu-Zn-based catalytic material and an MFI-type zeolite catalytic material supporting a noble metal, in which the MFI-type zeolite catalytic material contains P, and a ratio of mass (M_{P}) of P in the MFI-type zeolite catalytic material to mass (M2) of the MFI-type zeolite catalytic material is more than 0 mass% and less than 4.5 mass%.
[2] The catalyst for synthesizing liquefied petroleum gas as described in [1], in which the noble metal is only Pt.
[3] The catalyst for synthesizing liquefied petroleum gas as described in [1], in which the noble metal is only Pd.
[4] The catalyst for synthesizing liquefied petroleum gas as described in [1], in which the noble metal includes Pt and Pd.
[5] The catalyst for synthesizing liquefied petroleum gas as described in [4], in which a ratio (M_{Pd}/ (M_{Pt}+M_{Pd})) of mass (M_{Pd}) of Pd to total mass (M_{Pt}+M_{Pd}) of mass (M_{Pt}) of Pt and the mass (M_{Pd}) of Pd supported on the MFI-type zeolite catalytic material is 0.70 or less.
[6] The catalyst for synthesizing liquefied petroleum gas as described in any one of [1] to [5], in which a ratio of mass (M_{N}) of the noble metal in the MFI-type zeolite catalytic material to the mass (M2) of the MFI-type zeolite catalytic material is 0.1 mass% or more and 1.0 mass% or less.
[7] The catalyst for synthesizing liquefied petroleum gas as described in any one of [1] to [6], in which the Cu-Zn-based catalytic material and the MFI-type zeolite catalytic material exist independently from each other, and both of the Cu-Zn-based catalytic material and the MFI-type zeolite catalytic material are in the form of granulated powder or molded body.
[8] A method for producing liquefied petroleum gas including: a reduction treatment step of reducing the catalyst for synthesizing liquefied petroleum gas as described in any one of [1] to [7]; a supply step of supplying carbon monoxide and hydrogen to the catalyst for synthesizing liquefied petroleum gas reduced in the reduction treatment step; and a synthesis step of synthesizing liquefied petroleum gas by reacting the carbon monoxide and the hydrogen supplied in the supply step using the catalyst for synthesizing liquefied petroleum gas to be reduced.
[9] The method for producing liquefied petroleum gas as described in [8], in which a gas hourly space velocity (GHSV) for supplying the carbon monoxide and the hydrogen is 500/h or more and 20000/h or less in the supply step.
[10] The method for producing liquefied petroleum gas as described in [8] or [9], in which the carbon monoxide and the hydrogen are reacted at a temperature of 260°C or more and 330°C or less in the synthesis step.
[11] The method for producing liquefied petroleum gas as described in any one of [8] to [10], in which the carbon monoxide and the hydrogen are reacted under a pressure of 2.0 MPa or more and 6.0 MPa or less in the synthesis step.

### Effects of the Invention

According to the present disclosure, it is possible to provide a catalyst for synthesizing liquefied petroleum gas and a method for producing liquefied petroleum gas, which can increase the ratio of propane to the combined total of propane and butane in the produced liquefied petroleum gas.

### BRIEF DESCRIPTION OF THE DRAWINGS

Fig. 1 is a diagram illustrating the results of Examples and Comparative Examples.
Fig. 2 is a diagram illustrating the results of Examples and Comparative Examples.
Fig. 3 is a diagram illustrating the results of Examples and Comparative Examples.
Fig. 4 is a diagram illustrating the results of a long-term stability test in Example 2.

### PREFERRED MODE FOR CARRYING OUT THE INVENTION

A detailed description based on the embodiments follows.

The present inventors, after intensive research, found that by utilizing a catalyst for synthesizing liquefied petroleum gas including a Cu-Zn-based catalytic material and an MFI-type zeolite catalytic material supporting a noble metal, in which the MFI-type zeolite catalytic material contains P, and the ratio of the mass (M_{P}) of P in the MFI-type zeolite catalytic material to the mass (M2) of the MFI-type zeolite catalytic material is more than 0 mass% and less than 4.5 mass%, it is possible to increase the ratio of propane to the combined total of propane and butane in the produced liquefied petroleum gas.

The catalyst for synthesizing liquefied petroleum gas according to the present embodiment includes a Cu-Zn-based catalytic material and an MFI-type zeolite catalytic material supporting a noble metal (hereinafter, also simply referred to as the zeolite catalytic material), in which the MFI-type zeolite catalytic material contains P, and the ratio of the mass (M_{P}) of P in the MFI-type zeolite catalytic material to the mass (M2) of the MFI-type zeolite catalytic material is more than 0 mass% and less than 4.5 mass%.

As mentioned above, the catalyst for synthesizing liquefied petroleum gas of the embodiment includes a Cu-Zn-based catalytic material and an MFI-type zeolite catalytic material. The catalyst for synthesizing liquefied petroleum gas can synthesize liquefied petroleum gas from carbon monoxide and hydrogen. The liquefied petroleum gas synthesized by the catalyst for synthesizing liquefied petroleum gas of the present embodiment mainly consists of propane and butane, with propane being more predominant than butane. In the liquefied petroleum gas synthesized by the catalyst for synthesizing liquefied petroleum gas of the present embodiment, the ratio of the combined total of propane and butane to the liquefied petroleum gas is, for example, 20 Cmol% or more.

The Cu-Zn-based catalytic material composing the catalyst for synthesizing liquefied petroleum gas has the function as liquefied petroleum gas precursor synthesis catalyst to synthesize the liquefied petroleum gas precursors such as methanol and dimethyl ether from carbon monoxide and hydrogen.

The Cu-Zn-based catalytic material composing the catalyst for synthesizing liquefied petroleum gas is a catalyst including copper oxide and zinc oxide, and excels in performance of synthesizing liquefied petroleum gas precursors, among catalysts for synthesizing liquefied petroleum gas precursors. The Cu-Zn-based catalytic material may also include, in addition to copper oxide and zinc oxide, other component such as aluminum oxide, gallium oxide, zirconium oxide, or indium oxide. By including component such as aluminum oxide, gallium oxide, zirconium oxide, or indium oxide, the dispersion of copper oxide and zinc oxide can be improved. From the perspective of efficiently forming the number of interfaces between copper and zinc, which are believed to be active sites, it is preferable that the Cu-Zn-based catalytic material is a ternary oxide consisting of copper oxide, zinc oxide, and aluminum oxide.

The zeolite catalytic material composing the catalyst for synthesizing liquefied petroleum gas synthesizes liquefied petroleum gas from the liquefied petroleum gas precursors generated by the Cu-Zn-based catalytic material.

Regarding the zeolite catalytic material that composes the catalyst for synthesizing liquefied petroleum gas, the type of zeolite is MFI-type. The MFI-type zeolite catalytic material has a smaller pore diameter compared to beta-type zeolite, and it is assumed that propane can be synthesized more efficiently than butane among components of liquefied petroleum gas and the yield of propane can be enhanced.

Furthermore, the MFI-type zeolite catalytic material supports a noble metal. The MFI-type zeolite catalytic material supporting such noble metal can efficiently react the liquefied petroleum gas precursors, potentially leading to a higher yield of propane.

As the noble metal supported on the MFI-type zeolite catalytic material may include platinum group elements such as Pt (platinum), Pd (palladium), Rh (rhodium), and Ru (ruthenium). The noble metal can be single type or plural type. When the noble metal is plural type, there is no specific restriction on the state of the noble metals supported on the zeolite catalytic material; for example, the noble metals may coexist as individual metals, form alloy, or coexist as individual metals and alloy.

Regarding the ratio of the mass (M_{N}) of the noble metal in the MFI-type zeolite catalytic material to the mass (M2) of the MFI-type zeolite catalytic material, the lower limit is preferably 0.1 mass% or more, more preferably 0.2 mass% or more, and even more preferably 0.3 mass% or more, and the upper limit is preferably 1.0 mass% or less, more preferably 0.8 mass% or less, and even more preferably 0.7 mass% or less.

The noble metal supported on the MFI-type zeolite catalytic material may be Pt (platinum) only, Pd (palladium) only, or may include both Pt and Pd. In particular, from the perspective of enhancing the yield of propane and butane, the zeolite catalytic material preferably supports Pt and Pd, and more preferably supports only Pt. When both Pt and Pd are supported, as the states of Pt and Pd supported on the zeolite catalytic material, Pt and Pd may coexist as individual metals, Pt and Pd may form alloys, or at least one of the individual metals of Pt and Pd and the alloy of Pt and Pd may coexist.

When the zeolite catalytic material supports Pt and Pd, regarding the ratio (M_{Pd}/ (M_{Pt}+M_{Pd})) of the mass (M_{Pd}) of Pd to the total mass (M_{Pt}+M_{Pd}) of mass (M_{Pt}) of Pt and the mass (M_{Pd}) of Pd supported on the zeolite catalytic material (hereinafter also referred to as mass ratio (M_{Pd}/ (M_{Pt}+M_{Pd}))), the upper limit is preferably 0.70 or less, more preferably 0.60 or less, and even more preferably 0.50 or less. The lower limit of the mass ratio (M_{Pd}/ (M_{Pt}+M_{Pd})) is, for instance, 0.01 or more, preferably 0.15 or more, more preferably 0.20 or more, and even more preferably 0.25 or more. When the mass ratio (M_{Pd}/ (M_{Pt}+M_{Pd})) is 0.70 or less, the yield of butane can be increased.

Additionally, regarding the ratio of the total mass (M_{Pt}+M_{Pd}) of the mass (M_{Pt}) of Pt and the mass (M_{Pd}) of Pd in the zeolite catalytic material to the mass (M2) of the zeolite catalytic material, the lower limit is preferably 0.1 mass% or more, more preferably 0.2 mass% or more, and even more preferably 0.3 mass% or more, and the upper limit is preferably 1.0 mass% or less, more preferably 0.8 mass% or less, and even more preferably 0.7 mass% or less. When the zeolite catalytic material does not support Pd, the content ratio of Pd in the total content ratio is 0 (zero), which means that the total content ratio represents the content ratio of Pt.

When the ratio of the total mass (M_{Pt}+M_{Pd}) of the mass (M_{Pt}) of Pt and the mass (M_{Pd}) of Pd to the mass (M2) of the zeolite catalytic material is 0.1 mass% or more, liquefied petroleum gas can be synthesized efficiently. Moreover, when the ratio of the total mass (M_{Pt}+M_{Pd}) of the mass (M_{Pt}) of Pt and the mass (M_{Pd}) of Pd to the mass (M2) of the zeolite catalytic material is 1.0 mass% or less, efficient synthesis of liquefied petroleum gas can be maintained while suppressing the increase in material costs of Pt and Pd.

The presence or absence of the noble metal such as Pt or Pd supported on the zeolite catalytic material, the mass ratio (M_{Pd}/ (M_{Pt}+M_{Pd})), and the ratio of the total mass (M_{Pt}+M_{Pd}) of the mass (M_{Pt}) of Pt and the mass (M_{Pd}) of Pd to the mass (M2) of the zeolite catalytic material can be measured by ICP-OES (Inductively Coupled Plasma Optical Emission Spectroscopy).

In the present embodiment, the MFI-type zeolite catalytic material contains P (phosphorus), and the ratio of the mass (M_{P}) of P in the MFI-type zeolite catalytic material to the mass (M2) of the MFI-type zeolite catalytic material is more than 0 mass% and less than 4.5 mass%. Thus, when the ratio ((M_{P}/M2) × 100) (hereinafter also referred to as "content ratio of P") of the mass (M_{P}) of P in the MFI-type zeolite catalytic material to the mass (M2) of the MFI-type zeolite catalytic material is more than 0 mass% and less than 4.5 mass%, the ratio of propane to the combined total of propane and butane increases. In the present embodiment, the ratio of propane to the combined total of propane and butane in the produced liquefied petroleum gas ((molar number of propane / (molar number of propane + molar number of butane)) × 100) is, for example, 0.70 or more, and can further be 0.75 or more, or even 0.80 or more.

When the MFI-type zeolite catalytic material contains P, the acid sites (solid acid sites) of the zeolite catalytic material increase and simultaneously shift to weaker acid sites, thereby allowing for increasing the ratio of propane to the combined total of propane and butane. When the zeolite catalytic material contains P, it is presumed, as shown in the following formula (1), that P binds to the oxygen (O) associated with Si and the oxygen (O) associated with Al present on the surface of the zeolite catalytic material. However, when the ratio ((M_{P}/M2)×100) of the mass (M_{P}) of P in the MFI-type zeolite catalytic material to the mass (M2) of the MFI-type zeolite catalytic material is 4.5 mass% or more, the ratio of propane to the combined total of propane and butane decreases, and the yield of propane and the yield of butane also decrease.

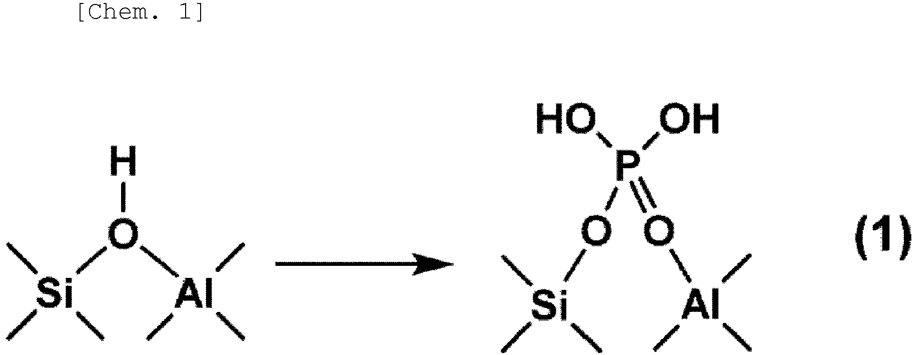

For the ratio ((M_{P}/M2) × 100) of P in the MFI-type zeolite catalytic material to the mass (M2) of the MFI-type zeolite catalytic material, the lower limit is more than 0 mass%, preferably 0.5 mass% or more, more preferably 1.0 mass% or more, and even more preferably 1.5 mass% or more, and the upper limit is less than 4.5 mass%, preferably 4.0 mass% or less, more preferably 3.0 mass% or less, and even more preferably 2.5 mass%.

The presence or absence of P in the zeolite catalytic material, and the content ratio of P can be measured by ICP-OES (Inductively Coupled Plasma Optical Emission Spectroscopy).

By using the catalyst for synthesizing liquefied petroleum gas of the present embodiment, it is possible to produce propane with high yield even at lower synthesis temperature (for instance, at 330°C or below) of liquefied petroleum gas.

In synthesizing liquefied petroleum gas, raising the synthesis temperature may increase the yield of liquefied petroleum gas such as propane. However, when using the Cu-Zn-based catalyst having superior performance in synthesizing liquefied petroleum gas precursors, there is a tendency to aggregate at higher temperature, causing notable temporal deactivation of the Cu-Zn-based catalyst, making stable synthesis of liquefied petroleum gas over extended periods difficulty. On the other hand, when the synthesis temperature is reduced (for example, to 330°C or below) to suppress the deactivation of the Cu-Zn-based catalyst due to such high temperature, the deactivation of the Cu-Zn-based catalyst can be suppressed, but the yield of liquefied petroleum gas such as propane will be reduced.

However, the catalyst for synthesizing liquefied petroleum gas of the present embodiment can produce propane with high yield even at lower synthesis temperature (for instance, at 330°C or below). Therefore, by using the catalyst for synthesizing liquefied petroleum gas of the present embodiment and synthesizing at lower temperature (for instance, at 330°C or below), propane can be produced at high yield, while also suppressing the deactivation of the catalyst due to high temperatures. The synthesis temperature refers to the temperature of the catalyst for synthesizing liquefied petroleum gas.

In the present embodiment, even at low synthesis temperature (for example, at 330°C or below), the yield of propane in the produced liquefied petroleum gas is for example 10 Cmol% or higher, and can be increased to 15 Cmol% or higher or even to 20 Cmol% or higher.

In addition, the yield of butane in the liquefied petroleum gas produced in the present embodiment is, for example, 5 Cmol% or more. Furthermore, the yield of propane and butane (the combined total of the yield of propane and the yield of butane) in the present embodiment is, for example, 20 Cmol% or more, and preferably 25 Cmol% or more.

Additionally, in the zeolite catalytic material, the ratio of the molar number of SiO₂ to the molar number of Al₂O₃ (molar number of SiO₂ / molar number of Al₂O₃) (hereinafter simply referred to as the "molar ratio (SiO₂/Al₂O₃)") is preferably 20 or more and 60 or less. The zeolite catalytic material is an aluminosilicate. By substituting some of the silicon atoms in the silicate forming the zeolite framework of the zeolite catalytic material with aluminum atoms, the aluminum atoms become acid sites; therefore, the zeolite catalytic material exhibits functionality as a solid acid.

When the molar ratio (SiO₂/Al₂O₃) is 60 or less, the acid sites of the zeolite catalytic material increase; this leads to an increase in the production amount of liquefied petroleum gas and also to an increase in the amount of propane contained in the liquefied petroleum gas due to efficient synthesis of propane. Also, when the molar ratio (SiO₂/Al₂O₃) is 20 or more, the zeolite catalytic material supporting the noble metal and containing P can be produced easily while maintaining a high liquefied petroleum gas production capability and a high propane synthesis capability.

From the perspective of ease of production of the zeolite catalytic material, the molar ratio (SiO₂/Al₂O₃) is preferably 20 or more, more preferably 25 or more, and even more preferably 30 or more. From the perspective of high catalytic performance, the molar ratio (SiO₂/Al₂O₃) is preferably 60 or less, more preferably 50 or less, and even more preferably 40 or less.

The molar ratio (SiO₂/Al₂O₃) can be measured by ICP-OES (Inductively Coupled Plasma Optical Emission Spectroscopy).

Furthermore, the solid acid amount of the zeolite catalytic material is preferably 0.6 mmol/g or more, more preferably 0.8 mmol/g or more. When the solid acid amount is 0.6 mmol/g or more, the zeolite catalytic material supporting the noble metal can be produced easily while maintaining the high liquefied petroleum gas production capability and the high propane synthesis capability.

The above-mentioned solid acid amount can be measured by NH₃-TPD (Ammonia Temperature-Programmed Desorption).

In the present embodiment, regarding the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material (hereafter also simply referred to as the mass ratio of the Cu-Zn-based catalytic material), the lower limit is preferably 0.30 or more, more preferably 0.35 or more, and even more preferably 0.40 or more, and the upper limit is preferably 0.95 or less, more preferably 0.70 or less, even more preferably 0.65 or less, and especially preferably 0.60 or less. When the mass ratio of the Cu-Zn-based catalytic material is 0.30 or more and 0.95 or less, liquefied petroleum gas can be efficiently synthesized from carbon monoxide and hydrogen.

The Cu-Zn-based catalytic material and the MFI-type zeolite catalytic material preferably exist independently from each other, and both of the Cu-Zn-based catalytic material and the MFI-type zeolite catalytic material are preferably in the form of powder or molded body. For the catalyst for synthesizing liquefied petroleum gas, the Cu-Zn-based catalytic material and the MFI-type zeolite catalytic material are preferably not integrated (indistinctly integrated). The state of the Cu-Zn-based catalytic material and the zeolite catalytic material may be in the form of granulated powder (powder. for example, particle size of 10⁻⁹ to 10⁻⁴ m) or granules having a particle size larger than the granulated powder; however, when the Cu-Zn-based catalytic material is a molded body containing Cu-Zn-based catalytic material, and the zeolite catalytic material is a molded body containing zeolite catalytic material, the yield of propane and butane can be further increased. In other words, the catalyst for synthesizing liquefied petroleum gas is preferably a mixture of the molded bodies containing the Cu-Zn-based catalytic material and the molded bodies containing the zeolite catalytic material.

For the molded body containing the Cu-Zn-based catalytic material, the content ratio of the Cu-Zn-based catalytic material contained in the molded body is preferably 80 mass% or more, more preferably 90 mass% or more, and even more preferably 95 mass% or more. When this content ratio is 80 mass% or more, the liquefied petroleum gas precursors can be efficiently synthesized from carbon monoxide and hydrogen. The content ratio of the Cu-Zn-based catalytic material contained in the molded body may be 100 mass%. Additionally, this content ratio is preferably 98 mass% or less, more preferably 96 mass% or less, and even more preferably 94 mass% or less. When this content ratio is 98 mass% or less, the moldability and the mechanical strength of the molded body can be improved while maintaining efficient synthesis of the liquefied petroleum gas precursors.

The molded body containing the Cu-Zn-based catalytic material may also include various additives to improve the moldability and the mechanical strength, in addition to the Cu-Zn-based catalytic material. Examples of such additives include molding binders such as graphite and carbon black.

For the molded body containing the zeolite catalytic material, the content ratio of the zeolite catalytic material contained in the molded body is preferably 70 mass% or more, more preferably 80 mass% or more, and even more preferably 90 mass% or more. When this content ratio is 70 mass% or more, liquefied petroleum gas can be efficiently synthesized from the liquefied petroleum gas precursors. The content ratio of the zeolite catalytic material contained in the molded body may be 100 mass%. Additionally, this content ratio is preferably 98 mass% or less, more preferably 96 mass% or less, and even more preferably 94 mass% or less. When this content ratio is 98 mass% or less, the moldability and the mechanical strength of the molded body can be improved while maintaining efficient synthesis of liquefied petroleum gas.

Similar to the molded body containing the Cu-Zn-based catalytic material, the molded body containing the zeolite catalytic material may also include various additives to improve the moldability and the mechanical strength, in addition to the zeolite catalytic material. Examples of such additives include molding binders such as various clay binders, alumina-based binder, and silica-based binder. Various clay binders include kaolin-based binder, bentonite-based binder, talc-based binder, pyrophyllite-based binder, molysite-based binder, vermiculite-based binder, montmorillonite-based binder, chlorite-based binder, halloysite-based binder, etc. To effectively improve the catalyst activity and suppress the formation of catalyst poisoning substance such as coke, the molding binder is preferably a silica-based binder.

The shape of the Cu-Zn-based catalytic material and the zeolite catalytic material is not limited in particular. When the Cu-Zn-based catalytic material or the zeolite catalytic material is in a molded body, shapes such as cylindrical, clover-like, ring-like, spherical, or multi-holed can be chosen, for instance. In the case of cylindrical or clover-like shaped molded body, such molded body is preferably an extrusion-molded body.

Regarding the particle size of the molded body containing the Cu-Zn-based catalytic material and the molded body containing the zeolite catalytic material, the lower limit is preferably 200 um or more, more preferably 300 um or more, and the upper limit is preferably 10 mm or less, more preferably 5 mm or less, and even more preferably 3 mm or less. When such particle size is 200 um or more, pressure loss within the reactor can be prevented. When such particle size is 10 mm or less, the contact efficiency between the reactant and the catalyst in the reactor can be enhanced. The particle size can be determined by the dry sieving test method.

Furthermore, concerning the bulk density of the molded body containing the Cu-Zn-based catalytic material and the molded body containing the zeolite catalytic material, the lower limit is preferably 0.5 g/cm³ or more, and the upper limit is preferably 1.5 g/cm³ or less, more preferably 1.0 g/cm³ or less. The bulk density can be determined using the sock loading bulk density measurement method with a measuring cylinder.

Next, the method for producing liquefied petroleum gas in the embodiment will be described.

The method for producing liquefied petroleum gas in the embodiment includes a reduction treatment step, a supply step, and a synthesis step.

In the reduction treatment step, the catalyst for synthesizing liquefied petroleum gas is reduced. Preferably, the catalyst for synthesizing liquefied petroleum gas is reduced with hydrogen.

In the supply step, performed after the reduction treatment step, carbon monoxide (CO) and hydrogen (H₂) are supplied to the catalyst for synthesizing liquefied petroleum gas reduced in the reduction treatment step. Carbon monoxide and hydrogen are both in gaseous form. Regarding the supply method, carbon monoxide and hydrogen may be supplied separately, or a mixed gas containing carbon monoxide and hydrogen, such as synthesis gas, may be supplied.

In the synthesis step, performed after the supply step, carbon monoxide and hydrogen supplied in the supply step are reacted using the reduced catalyst for synthesizing liquefied petroleum gas to produce liquefied petroleum gas. By using the catalyst for synthesizing liquefied petroleum gas to react carbon monoxide and hydrogen, it is possible to improve the ratio of propane to the combined total of propane and butane in the produced liquefied petroleum gas. Additionally, the catalyst for synthesizing liquefied petroleum gas of the present embodiment is resistant to deactivation, has excellent long-term stability, and can maintain good catalytic performance for a long period (for example, 70 hours or longer).

Regarding the gas hourly space velocity (GHSV) for supplying carbon monoxide and hydrogen in the supply step, the lower limit is preferably 500/h or more, more preferably 1000/h or more, and even more preferably 1500/h or more; the upper limit is preferably 20000/h or less, more preferably 10000/h or less, and even more preferably 5000/h or less.

When the gas hourly space velocity (GHSV) is 500/h or more, it is possible to efficiently produce liquefied petroleum gas from carbon monoxide and hydrogen. Additionally, when the gas hourly space velocity (GHSV) is 20000/h or less, it is possible to suppress the increase in the content ratio of unreacted substance such as carbon monoxide or hydrogen in the gas containing the produced liquefied petroleum gas obtained after synthesis.

Regarding the temperature of the catalyst (synthesis temperature) in the synthesis step, the lower limit is preferably 260°C or more, more preferably 270°C or more, and even more preferably 280°C or more; the upper limit is preferably 330°C or less, more preferably 325°C or less, and even more preferably 320°C or less.

In the synthesis step, by reacting carbon monoxide and hydrogen at a temperature of 260°C or more, it is possible to efficiently produce liquefied petroleum gas from carbon monoxide and hydrogen. Additionally, by reacting carbon monoxide and hydrogen at a temperature of 330°C or less in the synthesis step, it is possible to suppress the deterioration of the catalytic performance of the catalyst for synthesizing liquefied petroleum gas due to temperature. Also, by reacting carbon monoxide and hydrogen at a temperature of 330°C or less, it is possible to suppress the decrease in the yield due to excessive decomposition of the produced liquefied petroleum gas (e.g., decomposition from propane to ethane, or from ethane to methane).

Regarding the pressure in the synthesis step, the lower limit is preferably 2.0 MPa or more, more preferably 3.0 MPa or more, and even more preferably 3.5 MPa or more; the upper limit is preferably 6.0 MPa or less, more preferably 5.5 MPa or less, and even more preferably 5.0 MPa or less, under which carbon monoxide and hydrogen are reacted.

In the synthesis step, by reacting carbon monoxide and hydrogen at a pressure of 2.0 MPa or more, it is possible to efficiently produce liquefied petroleum gas from carbon monoxide and hydrogen. Furthermore, by reacting carbon monoxide and hydrogen at a pressure of 6.0 MPa or less in the synthesis step, it is possible to suppress the deterioration of the catalytic performance of the catalyst for synthesizing liquefied petroleum gas due to pressure.

The catalyst for synthesizing liquefied petroleum gas can be produced, for instance, by mixing the Cu-Zn-based catalytic material and the zeolite catalytic material. The composition, ratio, and state of the Cu-Zn-based catalytic material and the zeolite catalytic material can be appropriately set depending on the desired liquefied petroleum gas.

The aforementioned molar ratio (SiO₂/Al₂O₃) of the zeolite catalytic material can be controlled by the amount of aluminum source added during the synthesis of the zeolite catalytic material.

The amount of solid acid in the zeolite catalytic material can be controlled by, for example, the synthesis conditions (such as pH) during the synthesis of the zeolite catalytic material.

The method for supporting noble metal such as platinum or palladium on the zeolite catalytic material is not particularly limited, but such method includes the impregnation method, immersion method, and ion-exchange method.

When plural types of noble metals are supported on the zeolite catalytic material, it is preferable to simultaneously support the plural types of noble metals using an impregnating solution or an immersion solution containing the plural types of noble metals to be supported, rather than sequentially supporting each noble metal through separate impregnation or immersion. For example, when supporting platinum and palladium on the zeolite catalytic material, it is preferable to simultaneously support both platinum and palladium using an impregnating solution or an immersion solution containing platinum and palladium, rather than sequentially supporting each metal through separate impregnation or immersion.

A Compound containing a noble metal can be used as a starting material for the noble metal to be supported on the zeolite catalytic material. For example, for the starting material for platinum, chloroplatinic acid hexahydrate, dinitrodiamine platinum, dichlorotetraamine platinum, platinum oxide, and platinum chloride can be used. For the starting material for palladium, palladium chloride, palladium nitrate, dinitrodiamine palladium, palladium sulfate, and palladium oxide can be used.

After impregnating the zeolite catalytic material with a solution of a compound containing a noble metal, such as a chloroplatinic acid solution and palladium chloride solution, or after immersing the zeolite catalytic material in the solution of the compound containing the noble metal, calcining the impregnated or immersed zeolite catalytic material can efficiently highly disperse the noble metal in the zeolite catalytic material and easily control the amount of the noble metal supported on the zeolite catalytic material.

The concentration of the compound containing the noble metal in the solution may be set according to the amount of the noble metal to be supported. For instance, the concentration of the chloroplatinic acid hexahydrate solution is preferably 0.15 mass% or more and 3.50 mass% or less. The concentration of the palladium chloride solution is preferably 0.1 mass% or more and 2.5 mass% or less. The supporting amount of the noble metal can be controlled based on the concentration of the solution.

For sufficient penetration of the noble metal into the zeolite catalytic material, the impregnation or immersion time of the solution is preferably 10 minutes or more and 5 hours or less.

The calcination temperature of the zeolite catalytic material is preferably 300°C or more and 600°C or less, and the calcination time of the zeolite catalytic material is preferably 30 minutes or more and 300 minutes or less.

The method for supporting phosphorus on the zeolite catalytic material is not particularly limited, but for example, an impregnation or immersion method can be used.

Orthophosphoric acid or phosphate ester can be used as a starting material for phosphorus when supporting phosphorus on the zeolite catalytic material. For example, an aqueous solution of orthophosphoric acid or phosphate ester can be used as an impregnation or immersion liquid.

Regarding the impregnation or immersion method when supporting phosphorus, after impregnating the zeolite catalytic material with a solution of orthophosphoric acid or phosphate ester (hereinafter referred to as "phosphoric acid solution") or after immersing the zeolite catalytic material in a phosphoric acid solution, and then calcining the impregnated or immersed zeolite catalytic material, P can be efficiently incorporated into the zeolite catalytic material, and the amount of P contained in the zeolite catalytic material can be easily controlled.

The concentration of the phosphoric acid solution is preferably 2 mass% or more and 20 mass% or less.

For sufficient penetration of the phosphoric acid solution into the zeolite catalytic material, the impregnation or immersion time of the phosphoric acid solution is preferably 10 minutes or more and 5 hours or less.

The calcination temperature of the zeolite catalytic material is preferably 300°C or more and 600°C or less. The calcination time of the zeolite catalytic material is preferably 30 minutes or more and 300 minutes or less.

The concentration of the phosphoric acid solution and the impregnation or immersion time of the phosphoric acid solution can control the content ratio of P.

After incorporating P into the zeolite catalytic material, it is preferable to support the noble metal such as platinum or palladium on the zeolite catalytic material. Specifically, for instance, after impregnating or immersing the zeolite catalytic material with the phosphoric acid solution, and then calcining the impregnated or immersed zeolite catalytic material, it is preferable to impregnate or immerse this calcined zeolite catalytic material in a solution containing noble metal such as platinum or palladium, and then calcine the zeolite catalytic material impregnated or immersed in the solution containing noble metal such as platinum or palladium.

The liquefied petroleum gas produced using the aforementioned catalyst for synthesizing liquefied petroleum gas contains a significant amount of propane as its component, and can therefore be stably used as an ideal fuel even in cold climates.

While embodiments have been described above, the present invention is not limited to these embodiments but encompasses all modifications within the scope of the present disclosure and the claims, and can be modified in various ways within the scope of the present disclosure.

### EXAMPLES

Next, Examples and Comparative Examples will be described, but the present disclosure is not limited to these Examples.

### <Example 1>

### (Cu-Zn-based Catalytic material)

A Cu-Zn-based catalytic material, specifically a ternary oxide of copper oxide, zinc oxide, and aluminum oxide (product name: 45776 Copper based methanol synthesis catalyst, manufactured by Alpha Aesar) was used. This Cu-Zn-based catalytic material was pelletized under a pressure of 5 MPa using a tablet press to produce pellets with a diameter of 20 mm and a thickness of about 1 mm; these pellets were then crushed in a mortar, and the crushed samples were sieved using overlaid 300 um and 500 um mesh sieves. As a result, a molded body composed of Cu-Zn-based catalytic material with a particle size of 300-500 µm, granular shape, and a bulk density of 0.9 g/cm³ was obtained.

### (MFI-type Zeolite Catalytic Material)

12 g of MFI-type zeolite (ZSM-5, molar number of SiO₂ / molar number of Al₂O₃ = 40) was added to an agate mortar; an aqueous solution prepared by dissolving 0.3834 g of orthophosphoric acid in 7.2000 g of deionized water was added dropwise using a pipette while mixing with a pestle to ensure uniformity; the impregnation took about 1 hour. Afterwards, the sample was dried at 100°C for 10 hours, and then calcined under an air atmosphere by raising the temperature from room temperature to 500°C for 50 minutes and maintaining this temperature for 120 minutes.

10 g of the calcined MFI-type zeolite containing P was added to an agate mortar; and an aqueous solution prepared by dissolving 0.0419 g of chloroplatinic acid hexahydrate and 0.0574 g of palladium chloride in 7.5758 g of 10% hydrochloric acid was added dropwise using a pipette while mixing with a pestle to ensure uniformity; the impregnation took about 1 hour. Afterwards, the sample was dried at 100°C for 10 hours, and then calcined under an air atmosphere by raising the temperature from room temperature to 500°C for 50 minutes and maintaining this temperature for 120 minutes, resulting in an MFI-type zeolite containing P and supporting both Pt and Pd. The sample was pelletized using a tablet press to produce pellets with a diameter of 20 mm and a thickness of about 1 mm; the pellets were then crushed in a mortar, and the crushed samples were sieved using overlaid 300 um and 500 um mesh sieves. As a result, a molded body composed of the MFI-type zeolite catalytic material containing P and supporting both Pt and Pd, with a particle size of 300-500um, granular shape, and a bulk density of 0.8 g/cm³ was obtained.

### (Production of Liquefied Petroleum Gas)

For the catalyst for synthesizing liquefied petroleum gas, a mixture of the molded body made from the Cu-Zn-based catalytic material obtained as mentioned above and the molded body made from the MFI-type zeolite catalytic material obtained as mentioned above was used. The ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material is listed in Table 1. It should be noted that M2 represents the combined total of the mass of the supported noble metals (Pt, Pd), the mass of the MFI-type zeolite catalytic material supporting the noble metals, and the mass of P contained therein. Using the catalyst for synthesizing liquefied petroleum gas, liquefied petroleum gas was produced under the following conditions.

First, the catalyst for synthesizing liquefied petroleum gas was subjected to a reduction treatment with hydrogen. Then, carbon monoxide and hydrogen were supplied to the catalyst for synthesizing liquefied petroleum gas at a Gas Hourly Space Velocity (GHSV) of 2000/h. While supplying carbon monoxide and hydrogen to the catalyst for synthesizing liquefied petroleum gas, the temperature (synthesis temperature) was controlled at 320°C and the pressure at 5.0 MPa to produce liquefied petroleum gas from carbon monoxide and hydrogen.

For the reaction synthesizing liquefied petroleum gas from a mixed gas of carbon monoxide and hydrogen (CO:H₂ = 1:2 (molar ratio)), a fixed-bed high-pressure circulating reactor was used. The reactor employed was made of stainless steel (with an inner diameter of 6.2 mm and a total length of 60 cm). The catalyst was packed in the center of the reactor, sandwiched between layers of glass wool. The reactor was placed in an electric furnace, and the temperature in the electric furnace was measured by a thermocouple inserted into the center of the furnace and controlled by PID. The temperature of the catalyst was measured using a thermocouple inserted into the center of the catalyst layer inside the reactor. Note that the temperature of the catalyst is the synthesis temperature. The reduction treatment of the catalyst for synthesizing liquefied petroleum gas was carried out by supplying H₂ to the catalyst inside the reactor at a flow rate of 40 ml/min at 380°C for 2 hours before synthesizing.

### (Gas Composition Analysis)

At a predetermined point of time after starting the reaction, the gas was analyzed using a gas chromatograph connected online. The gas chromatograph used was GC-2014 (manufactured by Shimadzu Corporation). The analysis target and the conditions are described below.

### <Analysis Conditions>

Column: RT-Q-BOND
Temperature program:
   (i) 45°C (held for 30 min)
   (ii) Heated at 2°C/min to 175°C
   (iii) 175°C (held for 40 min)
Analysis time: 135 min.

### [Measurement and Evaluation]

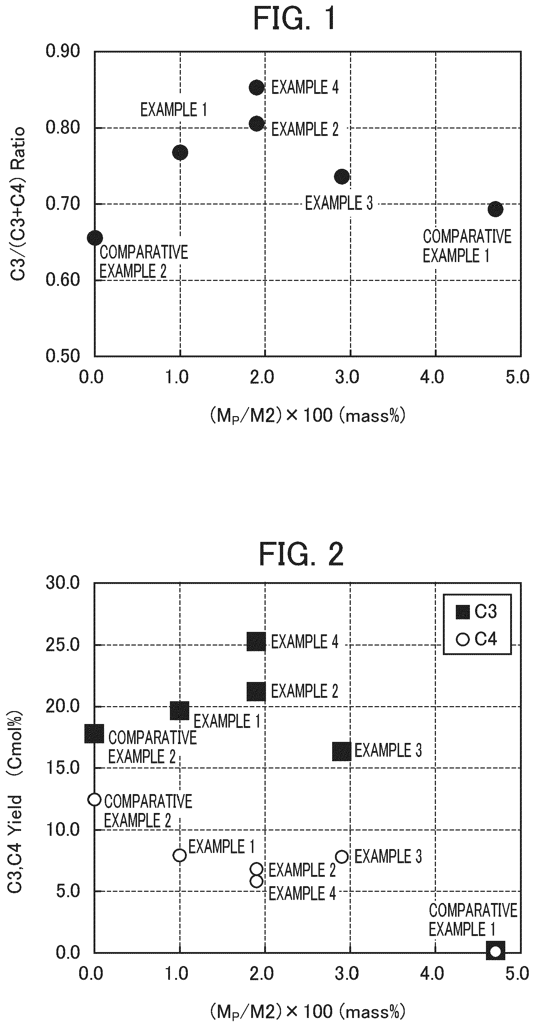

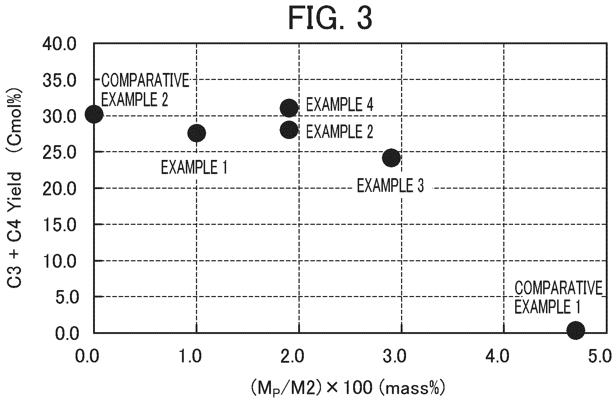

For the catalyst for synthesizing liquefied petroleum gas used in the Examples and Comparative Examples, and for the liquefied petroleum gas obtained in the above Examples and Comparative Examples, the following measurements and evaluations were performed. The results are illustrated in Table 1 and Figs. 1 to 3. Regarding the drawings, Fig. 1 illustrates the ratio of propane to the combined total of propane and butane, Fig. 2 illustrates the yield of propane and the yield of butane, and Fig. 3 illustrates the total yield of propane and butane. The results for the liquefied petroleum gas were measured 6 hours after the start of the reaction with carbon monoxide and hydrogen.

[1] Ratio (Molar Number of SiO₂ / Molar Number of Al₂O₃)
   The SiO₂/Al₂O₃ ratio (molar number of SiO₂ / molar number of Al₂O₃) was measured by ICP-OES (Inductively Coupled Plasma Optical Emission Spectroscopy).
[2] Presence or absence of Pd and Pt, Ratio (((M_{Pt}+M_{Pd})/ M2) × 100) of Total Mass (M_{Pt}+M_{Pd}) of Mass (M_{Pt}) of Pt and Mass (M_{Pd}) of Pd to Mass (M2) of MFI-type Zeolite Catalytic Material, Total Content Ratio of Pd and Pt (the total mass (M_{Pt}+M_{Pd}) of Mass (M_{Pt}) of Pt and Mass (M_{Pd}) of Pd to the mass (M2) of the zeolite catalytic material), and Ratio (M_{Pd}/ (M_{Pt}+M_{Pd})) of Mass (M_{Pd}) of Pd to Total Mass (M_{Pt}+M_{Pd}) of Mass (M_{Pt}) of Pt and Mass (M_{Pd}) of Pd
   The presence or absence of Pd and Pt supported on the MFI-type zeolite catalytic material, the ratio (((M_{Pt}+M_{Pd})/ M2) × 100), and the ratio (M_{Pd}/ (M_{Pt}+M_{Pd}) were measured by ICP-OES (Inductively Coupled Plasma Optical Emission Spectroscopy).
[3] Presence or absence of P, and Content Ratio of P (Ratio ((M_{P}/M2) × 100)) of Mass (M_{P}) of P in MFI-type Zeolite Catalytic Material to Mass (M2) of MFI-type Zeolite Catalytic Material)
   The presence or absence of P in the MFI-type zeolite catalytic material and the content ratio of P in the MFI-type zeolite catalytic material were measured by ICP-OES (Inductively Coupled Plasma Optical Emission Spectroscopy).
[4] CO Conversion Rate (CO Conversion) CO Convertion Rate (%) = [(CO flow rate at the inlet (µmol/min) - CO flow rate at the outlet (µmol/min)) / CO flow rate at the inlet (µmol/min)] x 100 The CO conversion rate indicates the ratio of carbon monoxide (CO) in the reaction raw gas being converted to hydrocarbons and the like.
[5] Propane Yield (C3 Yield as illustrated in the drawings) Propane Yield (Cmol%) = [(C3 Production Rate × 3) / (Inlet CO Flow Rate) × 106/22400] × 100 The unit of the C3 production rate is Cumol/min, and the unit of the inlet CO flow rate is ml (Normal)/min. C3 represents propane.
[6] Butane Yield (C4 Yield as illustrated in the drawings) Butane Yield (Cmol%) = [(C4 Production Rate × 4) / (Inlet CO Flow Rate) × 10622400] × 100 The unit of the C4 production rate is Cumol/min, and the unit of the inlet CO flow rate is ml (Normal)/min. C4 represents butane.
[7] Total Yield of Propane and Butane (C3+C4 Yield as illustrated in the drawings) Total Yield of Propane and Butane (Cmol%) = Yield of propane (Cmol%) + Yield of butane (Cmol%)
[8] Ratio of Propane to Combined Total of Propane and Butane (C3/(C3+C4) Ratio as illustrated in the drawings) Ratio of propane to the combined total of propane and butane = [molar number of propane / (molar number of propane + molar number of butane)] × 100

### <Example 2>

The operations were carried out in the same manner as in Example 1, except that an aqueous solution prepared by dissolving 0.7747 g of orthophosphoric acid in 7.2000 g of deionized water was used instead of the aqueous solution prepared by dissolving 0.3834 g of orthophosphoric acid in 7.2000 g of deionized water.

### <Example 3>

The operations were carried out in the same manner as in Example 1, except that an aqueous solution prepared by dissolving 1.1740 g of orthophosphoric acid in 7.2000 g of deionized water was used instead of the aqueous solution prepared by dissolving 0.3834 g of orthophosphoric acid in 7.2000 g of deionized water.

### <Example 4>

The operations were carried out in the same manner as in Example 1, except that a quaternary oxide CuO-ZnO-ZrO₂-Al₂O₃ of copper oxide, zinc oxide, zirconium oxide and aluminum oxide was used as the Cu-Zn-based catalytic material, instead of the ternary oxide of copper oxide, zinc oxide and aluminum oxide (product name: 45776 Copper based methanol synthesis catalyst, manufactured by Alpha Aesar), and an aqueous solution prepared by dissolving 0.1334 g of chloroplatinic acid hexahydrate in 7.5758 g of 10% hydrochloric acid was used as the MFI-type zeolite catalytic material, instead of the aqueous solution prepared by dissolving 0.0419 g of chloroplatinic acid hexahydrate and 0.0574 g of palladium chloride in 7.5758 g of 10% hydrochloric acid. CuO-ZnO-ZrO₂-Al₂O₃ was prepared according to the following procedure.

### (Preparation method of CuO-ZnO-ZrO₂-Al₂O₃)

95.13 g of copper nitrate trihydrate, 49.73 g of zinc nitrate hexahydrate, 19.33 g of aluminum nitrate nonahydrate, and 5.31 g of zirconyl nitrate dihydrate were dissolved in 584 g of distilled water to prepare Solution A. Additionally, 148 g of anhydrous sodium carbonate was dissolved in 2000 g of distilled water to prepare Solution B.

A 5000 ml separable flask with a stirrer was charged with 500 g of distilled water and heated in a water bath until the water reached 65°C. The entirety of Solution A and 900 ml of Solution B were added to the separable flask by setting the rate of a liquid delivery pump to complete the addition in 70 minutes. Vigorous stirring was maintained during the addition. The precipitate slurry temperature was adjusted to 65°C with the water bath, as needed. After the addition of Solution A and Solution B, the pH was approximately 5.6. The remaining portion of Solution B was then slowly added until the pH reached 6.5. After reaching pH 6.5, the precipitate slurry was stirred vigorously at 65°C for 2 hours. The pH after 2 hours was approximately 7.2.

Subsequently, the precipitation slurry was transferred to a suction filtration apparatus and filtered to obtain a precipitate cake. The obtained precipitate cake was washed 20 times with 250 ml of distilled water to remove Na ion from the precipitate cake.

After washing, the precipitate cake was transferred to an evaporating dish and dried in a drying oven at 120°C for 12 hours. It was then heated in a calcination furnace at a rate of 10°C/min up to 350°C and calcined at 350°C for 2 hours; the resulting calcined product was then thoroughly ground in an agate mortar to produce a powder. This powder was pelletized under a pressure of 5 MPa using a tablet press to produce pellets with a diameter of 20 mm and a thickness of about 1 mm, the pellets were then crushed in a mortar, and the crushed samples were sieved using overlaid 300 um and 500 um mesh sieves. As a result, a molded body composed of Cu-Zn-based catalytic material with a particle size of 300-500 µm, granular shape, and a bulk density of 0.9 g/cm³ was obtained.

The Cu-Zn-based catalytic material consisted of copper oxide (CuO), zinc oxide (ZnO), zirconium oxide (ZrO₂), and aluminum oxide (Al₂O₃), and had a chemical composition with 62.7 mass% copper oxide, 27.3 mass% zinc oxide, 5.0 mass% zirconium oxide, and 5.3 mass% aluminum oxide.

### <Comparative Example 1>

The operations were carried out in the same manner as in Example 1, except that an aqueous solution prepared by dissolving 1.9979 g of orthophosphoric acid in 7.2000 g of deionized water was used instead of the aqueous solution prepared by dissolving 0.3834 g of orthophosphoric acid in 7.2000 g of deionized water.

### <Comparative Example 2>

The operations were carried out in the same manner as in Example 1, except for adding 10 g of MFI-type zeolite (ZSM-5, molar number of SiO₂ / molar number of Al₂O₃ = 40) to an agate mortar, and adding dropwise an aqueous solution prepared by dissolving 0.0419 g of chloroplatinic acid hexahydrate and 0.0574 g of palladium chloride in 7.5758 g of 10% hydrochloric acid using a pipette, without the operations of impregnation of the aqueous solution prepared by dissolving 0.3834 g of orthophosphoric acid in 7.2000 g of deionized water, drying, and calcination.

**[Table 1]**

| | Zeolite catalytic material | | | | | M 1/(M 1 + M 2) |
|---|---|---|---|---|---|---|
| | (M_{P}/M2) ×100 (mass %) | Supported element | M_{Pd}/(M_{Pd}+M_{Pt}) | ((M_{Pd}+M_{Pd}/M2) ×100 (mass %) | Zeolite | |
| Example 1 | 1.0 | Pt, Pd | 0.67 | 0.50 | M F I | 0.50 |
| Example 2 | 1.9 | Pt, Pd | 0.67 | 0.50 | M F I | 0.50 |
| Example 3 | 2.9 | Pt, Pd | 0.67 | 0.50 | M F I | 0.50 |
| Example 4 | 1.9 | Pt | 0.00 | 0.50 | M F I | 0.50 |
| Comparative Example 1 | 4.7 | Pt, Pd | 0.67 | 0.50 | M F I | 0.50 |
| Comparative Example 2 | 0.0 | Pt, Pd | 0.67 | 0.50 | M F I | 0.50 |

As illustrated in Fig. 1, Examples 1 to 4, which include the Cu-Zn-based catalytic material and the MFI-type zeolite catalytic material supporting the noble metal, and the ratio of the mass (M_{P}) of P in the MFI-type zeolite catalytic material to the mass (M2) of the MFI-type zeolite catalytic material is more than 0 mass% and less than 4.5 mass%, had a notably higher ratio of propane to the combined total of propane and butane, as compared to Comparative Example 2 where the MFI-type zeolite catalytic material does not contain P, and Comparative Example 1 where the ratio of the mass (M_{P}) of P in the MFI-type zeolite catalytic material to the mass (M2) of the MFI-type zeolite catalytic material is 4.5 mass% or more. Additionally, in Examples 1 to 4 as illustrated in Figs. 2 and 3, despite the synthesis temperature being low and 320°C, the yield of propane was high, and the total yield of propane and butane was roughly the same as that in Comparative Example 2. The CO conversion rate was above 80% in both the Examples and Comparative Examples, indicating sufficient CO reactivity.

### (Long-term Stability Test)

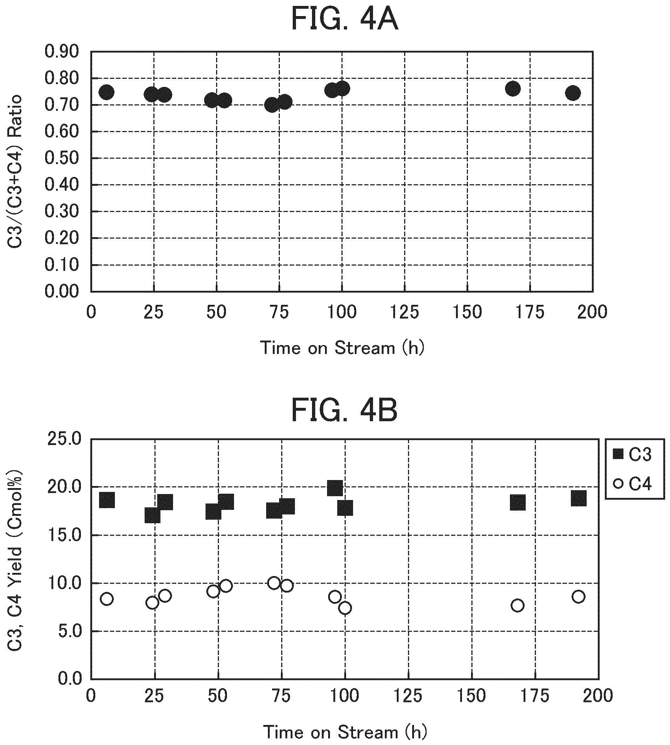

The operations were carried out in the same manner as in Example 2 except that the reaction temperature was changed to 300°C and the measurement was performed up to 200 hours after the start of reaction between carbon monoxide and hydrogen, and the results obtained from the measurement are illustrated in Fig. 4.

From Fig. 4, it can be observed that the catalyst for synthesizing liquefied petroleum gas of the present invention, which includes the Cu-Zn-based catalytic material and the MFI-type zeolite catalytic material supporting the noble metal, in which the ratio of the mass (M_{P}) of P in the MFI-type zeolite catalytic material to the mass (M2) of the MFI-type zeolite catalytic material is more than 0 mass% and less than 4.5 mass%, is less prone to deactivation at the lower synthesis temperature of around 300°C and demonstrates long-term stable functionality.

## Claims

1. A catalyst for synthesizing liquefied petroleum gas, the catalyst comprising:
a Cu-Zn-based catalytic material; and
an MFI-type zeolite catalytic material supporting a noble metal,
wherein the MFI-type zeolite catalytic material contains P, and
a ratio of mass (M_{P}) of P in the MFI-type zeolite catalytic material to mass (M2) of the MFI-type zeolite catalytic material is more than 0 mass% and less than 4.5 mass%.

2. The catalyst for synthesizing liquefied petroleum gas according to claim 1, wherein the noble metal is only Pt.

3. The catalyst for synthesizing liquefied petroleum gas according to claim 1, wherein the noble metal is only Pd.

4. The catalyst for synthesizing liquefied petroleum gas according to claim 1, wherein the noble metal includes Pt and Pd.

5. The catalyst for synthesizing liquefied petroleum gas according to claim 4, wherein a ratio (M_{Pd}/ (M_{Pt}+M_{Pd})) of mass (M_{Pd}) of Pd to total mass (M_{Pt}+M_{Pd}) of mass (M_{Pt}) of Pt and the mass (M_{Pd}) of Pd supported on the MFI-type zeolite catalytic material is 0.70 or less.

6. The catalyst for synthesizing liquefied petroleum gas according to any one of claims 1 to 5, wherein a ratio of mass (M_{N}) of the noble metal in the MFI-type zeolite catalytic material to the mass (M2) of the MFI-type zeolite catalytic material is 0.1 mass% or more and 1.0 mass% or less.

7. The catalyst for synthesizing liquefied petroleum gas according to any one of claims 1 to 6, wherein
the Cu-Zn-based catalytic material and the MFI-type zeolite catalytic material exist independently from each other, and
both of the Cu-Zn-based catalytic material and the MFI-type zeolite catalytic material are in the form of granulated powder or molded body.

8. A method for producing liquefied petroleum gas, the method comprising:
a reduction treatment step of reducing the catalyst for synthesizing liquefied petroleum gas according to any one of claims 1 to 7;
a supply step of supplying carbon monoxide and hydrogen to the catalyst for synthesizing liquefied petroleum gas reduced in the reduction treatment step; and
a synthesis step of synthesizing liquefied petroleum gas by reacting the carbon monoxide and the hydrogen supplied in the supply step using the catalyst for synthesizing liquefied petroleum gas to be reduced.

9. The method for producing liquefied petroleum gas according to claim 8, wherein a gas hourly space velocity (GHSV) for supplying the carbon monoxide and the hydrogen is 500/h or more and 20000/h or less in the supply step.

10. The method for producing liquefied petroleum gas according to claim 8 or 9, wherein the carbon monoxide and the hydrogen are reacted at a temperature of 260°C or more and 330°C or less in the synthesis step.

11. The method for producing liquefied petroleum gas according to any one of claims 8 to 10, wherein the carbon monoxide and the hydrogen are reacted under a pressure of 2.0 MPa or more and 6.0 MPa or less in the synthesis step.
